(19) Europäisches Patentamt
European Patent Office
Office européen des brevets

(11) Publication number : **0 516 347 A1**

(12) # EUROPEAN PATENT APPLICATION

(21) Application number : **92304645.2**

(22) Date of filing : **21.05.92**

(51) Int. Cl.$^5$ : **C07D 498/22,** C07D 519/00, A61K 31/445, // (C07D498/22, 321:00, 311:00, 273:00, 221:00), (C07D519/00, 498:00, 498:00)

(30) Priority : **29.05.91 US 706811**
**29.05.91 US 706821**

(43) Date of publication of application :
**02.12.92 Bulletin 92/49**

(84) Designated Contracting States :
**AT BE CH DE DK ES FR GB GR IT LI LU NL PT SE**

(71) Applicant : **AMERICAN HOME PRODUCTS CORPORATION**
**685, Third Avenue**
**New York, New York 10017 (US)**

(72) Inventor : **Kao, Wenling**
**1742 Jennings Way**
**Paoli, Chester County, Pennsylvania (US)**
Inventor : **Vogel, Robert Lewis**
**20 Sleepy Hollow Road**
**Stratford, Camden County, Pennsylvania (US)**
Inventor : **Musser, John Henry**
**c/o Glyco Med. Inc., 860 Atlantic Avenue**
**Alameda County, California (US)**

(74) Representative : **Wileman, David Francis Dr. et al**
**c/o Wyeth Laboratories Huntercombe Lane South**
**Taplow Maidenhead Berkshire SL6 OPH (GB)**

(54) **Rapamycin derivatives.**

(57) The invention concerns a rapamycin compound of formula

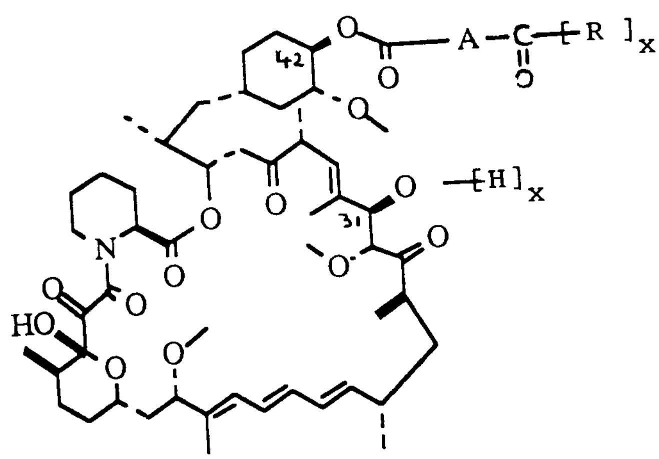

(I)

wherein x is zero or l ;
and when x is zero the

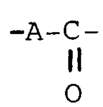

group is bonded to the oxygen attached to position 31 and when x is l, R represent a group

EP 0 516 347 A1

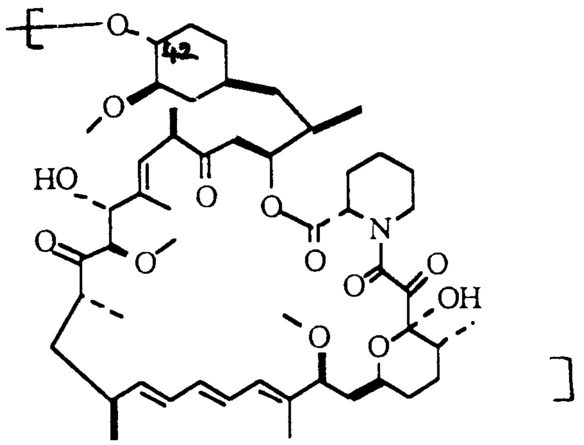

;

A represents

$-(CH_2)_n-$, $-(CH_2)_n-(CH=CH)-(CH_2)_m-$,
$-(CH_2)_n-(-C\equiv C-)-(CH_2)_m-$,

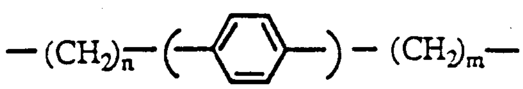

wherein n and m independently represent 1 to 10 providing that when x is O then neither n nor m is I, and further when x is I,A also represents

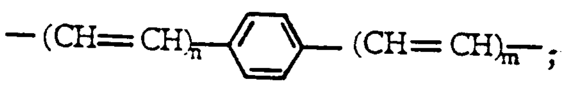

or a pharmaceutically acceptable salt thereof, which is by virtue of their immunosuppressive activity are useful in treating transplantation rejection host vs. graft disease, autoimmune diseases, and diseases of inflammation.

This invention relates to rapamycin derivatives, in particular rapamycin dimers and bicyclic rapamycin derivatives,their preparation, compositions containing them and a method of using them in the treatment of transplantation rejection, host vs. graft disease, autoimmune diseases, diseases of inflammation, solid tumors and fungal infenctions.

Rapamycin is a macrocyclic triene antibiotic produced by Streptomyces hygroscopicus, which was found to have antifungal activity, particularly against Candida albicans, both in vitro and in vivo [C. Vezina et al., J. Antibiot. 28, 721 (1975); S.N. Seghal et al., J. Antibiot. 28, 727 (1975); H. A. Baker et al., J. Antibiot. 31, 539 (1978); U.S. Patent 3,922,992; and U.S. Patent 3,993,749].

Rapamycin alone (U.S. Patent 4,885,171) or in combination with picibanil (U.S. Patent 4,401,653) has been shown to have antitumor activity. R. Martel et al. [Can. J. Physiol. Pharmacol. 55, 48 (1976) disclosed that rapamycin is effective in the experimental allergic encephalomyelitis model, a model for multiple sclerosis; in the adjuvant arthritis model, a model for rheumatoid arthritis; and effectively inhibited the formation of IGE-like antibodies.

The immunosuppressive effects of rapamycin have been disclosed in FASEB 3, 3411 ( 1989), rapamycin has been shown to be effective in inhibiting transplant rejection (U.S. Patent Application Ser. No. 362,544 filed June 6, 1989). Cyclosporin A and FK-506, other macrocyclic molecules, also have been shown to be effective as immunosuppressive agents, therefore useful in preventing transplant rejection [FASEB 3, 3411 (1989); FASEB 3, 5256 (1989); and R. Y. Calne et al., Lancet 1183 (1978).

Mono- and diacylated derivatives of rapamycin (esterified at the 28 and 43 positions) have been shown to be useful as antifungal agents (U.S. Patent 4,316,885) and used to make water soluble prodrugs of rapamycin (U.S. Patent 4,650,803). Recently, the numbering convention for rapamycin has been changed; therefore according to Chemical Abstracts nomenclature, the esters described above would be at the 31- and 42- positions.

This invention relates to rapamycin dimers of general formula (1), which possess immunosuppressive and/or antifungal and/or antitumor and/or antiinflammatory activity in vivo and/or inhibit thymocyte proliferation in vitro and are therefore useful in the treatment of transplantation rejection, autoimmune diseases (i.e. lupus, rheumatoid arthritis, diabetes mellitus, multiple sclerosis), fungal infections (i.e. Candida albicans), cancer, and diseases of inflammation.

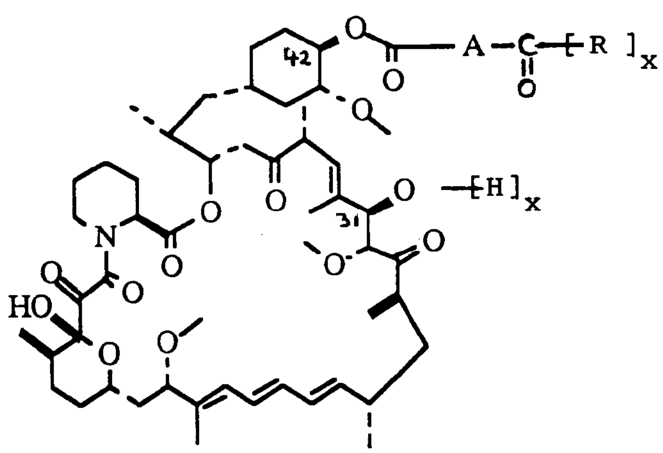

(I)

wherein x is zero or 1, and when x is zero the

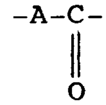

group is bonded to the oxygen attached to position 31, and when x is 1, [R] represents a group

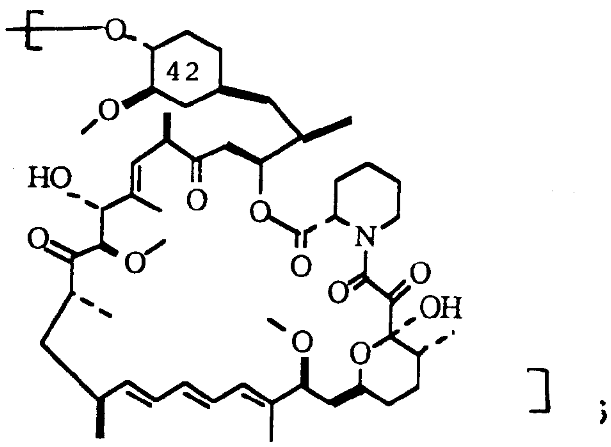

A represents

$-(CH_2)_n-$, $-(CH_2)_n-(CH{=}CH)-(CH_2)_m-$,
$-(CH_2)_n-(-C{\equiv}C-)-(-CH_2)_m-$,

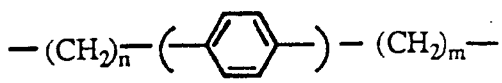

wherein n and m independently represent 1 to 10 providing that when x is O then neither n nor m is l, and further when x is l,A also represents

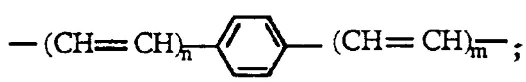

or a pharmaceutically acceptable salt thereof.

Processes for preparing the rapamycin derivatives of formula I are also within the scope of this invention.

The rapamycin derivatives of formula I are prepared by a process which comprises reacting rapamycin with a diacyl halide of formula II,

X-CO-A-COX (II)

wherein X is a halide, e.g chlorine and A is as defined above. Examples of A are $-(CH_2)_4-$, $-(CH_2)_5$, $-(CH_2)_6-$, $-(CH_2)_7-$ and

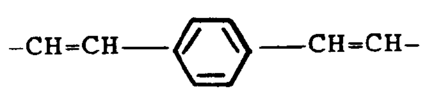

Formation of dimers of formula I where x is I is favoured relative to the bicyclic derivatives where x is O by increasing the ratio of rapamycin to diacyl halide reactant.

The rapamycin dimers (1) of this invention can be prepared by standard literature procedure as outlined below

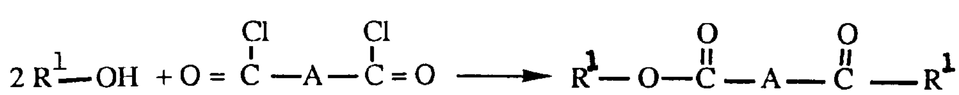

wherein $R^1$ is rapamycin and A is as defined above.

The bicyclic rapamycins of general formula (1) of this invention are preferably synthesized by reaction of rapamycin with diacyl halides at elevated temperature (30-70°C) in pyridine.

Rapamycin +

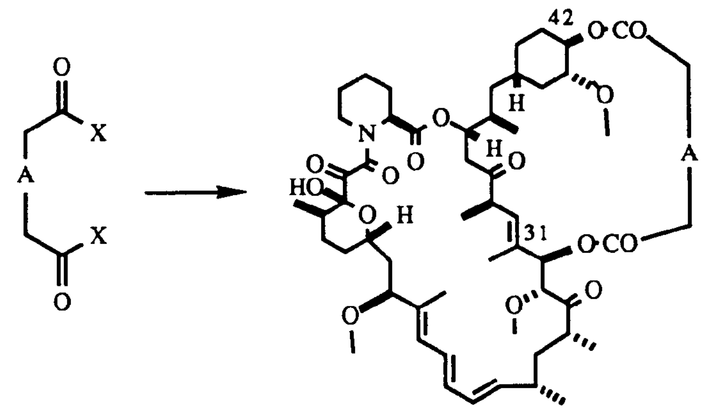

where X is halogen and A is as defined above.

The pharmaceutically acceptable salts may be formed from inorganic cations such as sodium, potassium, and the like.

The ester formation between alcohol and acyl halide has been described [Jerry March, Advanced Organic Chemistry, 3rd edition, published in 1985, page 346]. The specific reaction condition employed in this invention was developed by S. Rakhit of Ayerst Laboratories and reported in U.S. Patent 4,316,885 (February 23, 1982).

Immunosuppresive activity of the compounds of the present invention was evaluated in an in vitro standard pharmacological test procedure to measure lymphocyte proliferation (LAF).

The comitogen-induced thymocyte proliferation procedure (LAF) was used as an in vitro measure of the immunosuppressive effects of representative compounds. Briefly, cells from the thymus of normal BALB/c mice were cultured for 72 hours with PHA and IL-1 and pulsed with tritiated thymidine during the last six hours. Cells are cultured with and without various concentrations of rapamycin, cyclosporin A, or test compound. Cells are harvested and incorporated; radioactivity is determined. Inhibition of lymphoproliferation is assessed in percent change in counts per minute from non-drug treated controls. The results are expressed by the following ratio:

$$\frac{{}^3H - \text{control thymus cells} - H^3 - \text{rapamycin} - \text{treated thymus cells}}{{}^3H - \text{control thymus cells} - H^3 - \text{test compound} - \text{treated cells}}$$

The following table summarises the results of representative compounds of this invention in the standard test procedure.

TABLE 1

Biological Activity - LAF Assay

| | R/*A at 100 nM | at 10 nM | at $IC_{50}$ |
|---|---|---|---|
| Example 1 | 1.0 | 0.95 | - |
| Example 2 | 0.96 | 0.28 | - |
| Example 3 | 1.0 | 0.59 | - |
| Example 4 | 1.0 | 1.10 | 1.32 |
| Example 6 | 0.1 | 0.03 | - |
| Example 7 | 0.45 | 0.06 | - |
| Example 8 | 0.46 | 0.03 | - |

* Relative potency of analogs/rapamycin at dosages 100 nM and at 10 nM.

The results of this standard pharmacological test procedure for a representative compound of this invention demonstrates that the compounds of this invention are useful as immunosuppressive agents.

The compounds may be administered neat or with a pharmaceutical carrier to a mammal in need thereof. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellent.

Liquid pharmaceutical compositions which are sterile solutions or suspensions can be utilized by, for example, intramuscular, intraperitoneal or subcutaneous injection. Sterile solutions can also be administered intravenously. The compound can also be administered orally either in liquid or solid composition form.

Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form. The dosage to be used in the treatment must be subjectively determined by the attending physician.

Accordingly this invention provides a pharmaceutical composition comprising a compound of formula I or a pharmaceutically acceptable salt thereof and a pharmaceutically acceptable carrier.

The following examples illustrate the preparation of representative compounds of this invention.

## Example 1

Rapamycin-42,42′-diester with hexanedioic acid

A solution of 0. 13 g adipoyl chloride in 1 mL dry toluene was added dropwise at room temperature to a solution of 1. 10 g rapamycin in 20 mL dry toluene and 2 mL dry pyridine; the resulting solution was heated at 50°C under nitrogen with stirring for 65 hours. The product was extracted into ethyl acetate after addition of 20 mL 2N HCl and 20 mL brine. The ethyl acetate solution was dried over $MgSO_4$ and the solvent removed under reduced pressure. Chromatography through silica gel using 10% ethyl acetate in dichloromethane yielded 50 mg product as a yellow solid, mp 111-142°C. IR (KBr): 3430, 2925, 1718, 1645, 1352, 1170, 785, and 632 $cm^{-1}$. NMR ($CDCl_3$, 400 MHz): $\delta$ 3.36 (s, 6H, $OCH_3$), 3.33 (s, 6H, $OCH_3$), 3.14 (s, 6H, $OCH_3$), 1.75 (s, 6H, $CH_3$), 1.65 (s, 6H, $CH_3$). MS (neg FAB): 1937, 590.

## Example 2

Rapamycin-42,42′-diester with heptanedioic acid

A solution of 0.25 g pimeloyl chloride in 1 mL toluene was added to a solution of 1.12 g rapamycin in 35 mL toluene and 2 mL pyridine; the resulting solution was heated at 50°C for 44 hours under nitrogen with stirring. Upon cooling, 10 mL 2N HCl and 20 mL brine were added and the product was extracted into ethyl acetate (30 mL), which was washed with brine dried over $MgSO_4$ and evaporated. The residue was chromatographed through silica gel using a gradient of 5% to 30% ethyl acetate in dichloromethane, yielding 75 mg purified product as a pale yellow solid, mp 120- 150°C.

IR (KBr): 3440, 2930, 1732, 1648 and 1455 $cm^{-1}$. NMR ($CDCl_3$, 400 MHz): $\delta$ 3.37 (s, 6H, $OCH_3$), 3.34 (s, 6H, $OCH_3$), 3.14 (s, 6H, $OCH_3$). MS (neg FAB): 1951, 590.

**Example 3**

Rapamycin-42,42′-diester with octanedioic acid

A stirred solution of 1.24 g rapamycin and 0.30 g suberoyl chloride in 100 mL toluene and 2 mL pyridine was heated at 50°C for 66 hours under nitrogen, then cooled, diluted with 10 mL ethyl acetate and treated with 20 mL 2N HCl and 50 mL brine. The organic portion was washed with brine, dried over $MgSO_4$, stripped of solvent, and chromatographed through silica gel using a gradient of 0.5% to 20% methanol in dichloromethane, yielding 140 mg product as a pale yellow solid, mp 117-134°C.
IR (KBr): 3430, 2920, 1728, 1640, 1442 and 980 $cm^{-1}$. NMR ($CDCl_3$, 400 MHz): δ 3.37 (s, 6H, OMe), 3.33 (s, 6H, OMe), 3.14 (s, 6H, OMe). MS (neg FAB): 1965, 590.

**Example 4**

Rapamycin-42,42′-diacid with nonanedioic acid

A solution of 1.27 rapamycin and 50 mg azelaoyl chloride in 125 mL toluene and 2 mL pyridine was stirred at 50°C under nitrogen for 65 hours, then cooled and treated with 20 mL 2N HCl. The organic portion was washed with brine, dried over $MgSO_4$, stripped of solvent, and chromatographed through silica gel using a gradient of 0.5% to 10% methanol in dichloromethane, yielding 110 mg product as a pale yellow solid, mp 107- 125°C.
IR (KBr): 3450, 2935, 1730, 1650, 1455, 1100 and 990 $cm^{-1}$. NMR ($CDCl_3$, 400 MHz): δ 3.375 (s, 6H, OMe), 3.33 (s, 6H, OMe), 3.14 (s, 6H, OMe). MS (neg FAB): 1979, 590.

**Example 5**

Rapamycin 42,42′-diester with 1,4-phenylenediacrylic acid

A solution of 100 mg 1,4-phenylenediacrylic acid in 5 mL thionyl chloride was heated at reflux under nitrogen for two hours. The thionyl chloride was removed under reduced pressure; the residue was dissolved in 5 mL toluene, added to a stirred solution of 0.90 g rapamycin in 25 mL toluene and 2 mL pyridine, and heated at 50°C for 72 hours. The cooled reaction mixture was treated with 20 mL 2N HCl and diluted with 20 mL ethyl acetate and 50 mL brine. The product was extracted into ethyl acetate and chromatographed through silica gel using a gradient of 0 to 3 percent methanol in dichloromethane, yielding 60 mg product as a pale yellow solid, mp 121-131°C.
IR (KBr): 3420, 2930, 1715, 1640, 1445, 1100 and 980 $cm^{-1}$. NMR ($CDCl_3$, 400 MHz): δ 7.64 (d, 2H, J=12.0 Hz), 7.52 (s, 4H, aromatic), 6.47 (d, 2H, J=12.0 Hz), 3.38 (s, 6H, OMe), 3.32 (s, 6H, OMe), 3.12 (s, 6H, OMe). MS (neg FAB): 2009 (M-), 1112, 590.

**Example 6**

Rapamycin-31,42-cyclic diester with heptanedioic acid

A solution of 2.0 g pimeloyl chloride in 2 mL toluene was added to a solution of 5.0 g rapamycin in 250 mL dry toluene and 5 mL pyridine, and the resulting mixture was heated at 50-55°C under nitrogen for 65 hours, then cooled to ambient temperature, diluted with 100 mL ethyl acetate and treated with 50 mL 2N HCl and 200 mL brine. The organic portion was washed with brine, dried over $MgSO_4$ and stripped of solvent. Chromatography through silica gel using a gradient of 0.5% to 10% methanol in dichloromethane yielded an early fraction (200 mg) that contained desired product and a byproduct (M.S.). Further chromatography of that fraction on silica gel using a gradient of 20% to 30% ethyl acetate in dichloromethane yielded 40 mg of the title compound as a light tan solid, mp 107-121°C.
IR (KBr): 3420, 2930, 1735, 1647, 1460 and 990 $cm^{-1}$. NMR ($CDCl_3$, 400 MHz): δ 3.38 to 3.34 (broad, 6H, two OMe's), 316 (3H OMe). MS (neg FAB): 1037 (M-).

**Example 7**

Rapamycin-31,42-cyclic diester with hexanedioic acid

Adipoyl chloride (0.80 g) was added to a solution of 2.0 rapamycin in 50 mL toluene and 1 mL pyridine and

heated at 50°C under nitrogen for 90 hours. The reaction mixture was cooled to ambient temperature, diluted with 50 mL ethyl acetate, and treated with 20 mL 2N HCl and 50 mL brine. The aqueous portion was extracted with ethyl acetate; the organic portion was dried over $MgSO_4$ and stripped to a yellow-brown solid foam. Chromatography through silica gel beginning with dichloromethane followed by a methanol gradient of 0.5% to 3% in dichloromethane yielded 50 mg product as a yellow solid, mp 105-115°C.
IR (KBr): 3430, 2930, 1742, 1658, 1460 and 988 $cm^{-}1$. NMR ($CDCl_3$, 400 MHz): δ 3.31 (broad, 6H, two OMe's), 3.11 (3H, OMe). MS (neg FAB): 1023 (M-).

**Example 8**

Rapamycin-31,42-cyclic diester with octanedioic acid

Suberoyl chloride (0.80 g) was added to a solution of 2.0 rapamycim in 50 mL toluene and 1 mL pyridine and heated at 50°C under nitrogen for 92 hours. The reaction mixture was cooled to ambient temperature, diluted with 50 mL ethyl acetate, and treated with 20 mL 2N HCl and 50 mL brine. The aqueous portion was extracted with ethyl acetate; the organic portion was dried over $MgSO_4$ and stripped to a yellow-brown solid foam. Chromatography through silica gel beginning with dichloromethane followed by a methanol gradient of 0.5% to 3% in dichloromethane yielded 110 mg product as a pale yellow solid, mp 86-99°C.
IR (KBr): 3430, 2930, 1730, 1450 and 990 $cm^{-1}$. NMR ($CDCl_3$, 400 MHz): δ 3.32-3.29 (broad, 6H, two OMe's), 3.10 (3H, OMe). MS (neg FAB): 1051 (M-).

## Claims

**1.** A rapamycin compound of formula

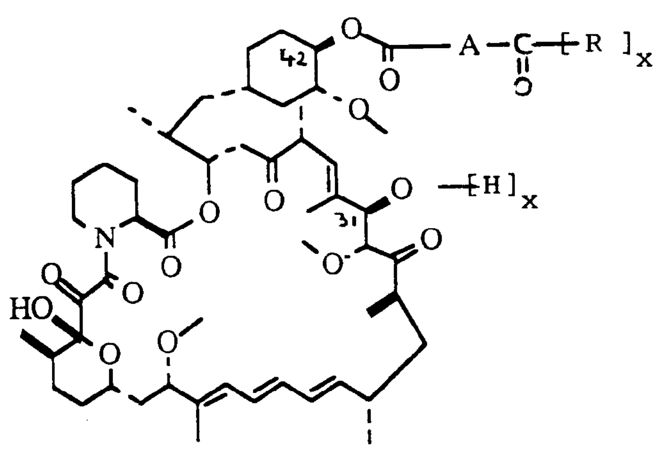

(I)

wherein x is zero or l;
and when x is zero the

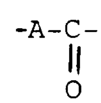

group is bonded to the oxygen attached to position 31 and when x is l, R represent a group

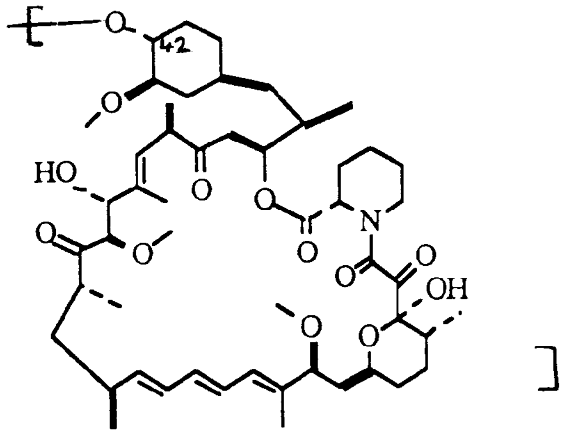 ;

A represents

$-(CH_2)_n-$, $-(CH_2)_n-(CH=CH)-(CH_2)_m-$,
$-(CH_2)_n-(-C\equiv C-)-(CH_2)_m-$,

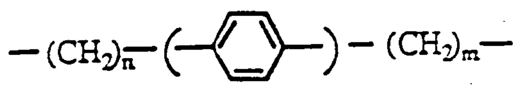

wherein n and m independently represent 1 to 10 providing that when x is 0 then neither n nor m is l, and further when x is l,A also represents

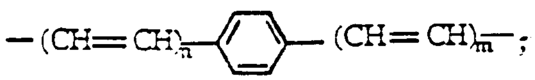

or a pharmaceutically acceptable salt thereof.

**2.** A compound as claimed in Claim 1 wherein A is $-(CH_2)_n-$ wherein n is 4 to 7 or A is

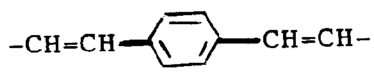

**3.** A compound as claimed in Claim 1 wherein x is O and A is $-(CH_2)_4-$.
**4.** A compound as claimed in Claim 1 wherein x is O and A is $-(CH_2)_5-$.
**5.** A compound as claimed in Claim 1 wherein x is O and A is $-(CH_2)_6-$.
**6.** A compound as claimed in Claim 1 wherein x is I and A is $-(CH_2)_4-$.
**7.** A compound as claimed in Claim 1 wherein x is I and A is $-(CH_2)_5-$.
**8.** A compound as claimed in Claim 1 wherein x is I and A is $-(CH_2)_6-$.
**9.** A compound as claimed in Claim 1 wherein x is I and A is $-(CH_2)_7-$.
**10** A compound as claimed in Claim 1 wherein x is I and A is

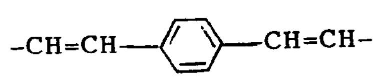

**11.** A process for preparing a compound of formula I as claimed in Claim 1 which comprises reacting rapamycin with a diacyl halide of formula II

X-CO-A-COX (II)

wherein X is a halide and A is as defined in Claim 1 and if desired isolating the product as a pharmaceutically acceptable salt thereof.

**12.** A process as claimed in Claim 11 wherein X is chlorine.

**13.** A pharmaceutical composition comprising a compound of formula I as claimed in Claim 1 and a phar-

maceutically acceptable carrier.

**Claims for the following Contracting States : GR, ES**

**1.** A process for preparing a rapamycin compound of formula

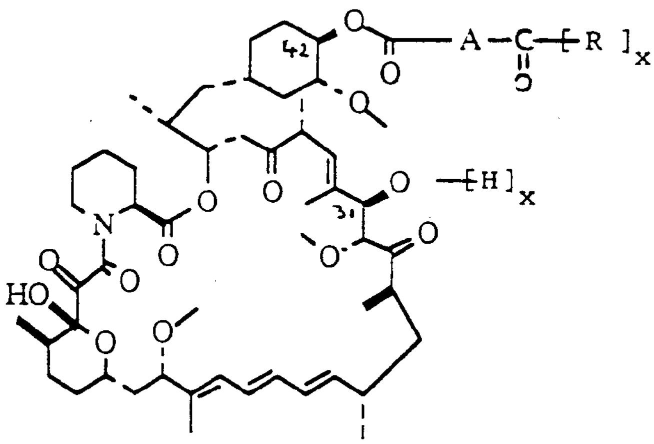

(I)

wherein x is zero or l;
and when x is zero the

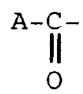

group is bonded to the oxygen attached to position 31 and when x is l, R represent a group

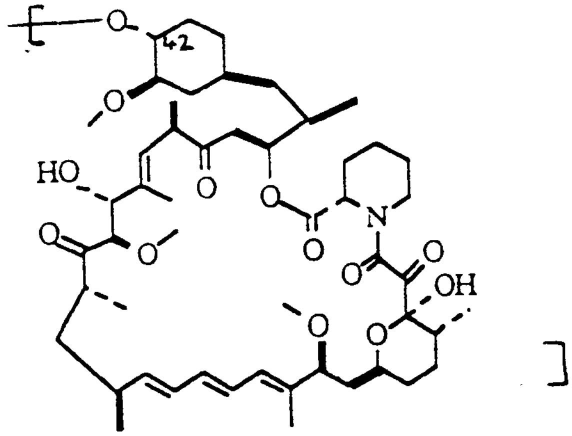
;

A represents

$-(CH_2)_n-$, $-(CH_2)_n-(CH=CH)-(CH_2)_m-$,
$-(CH_2)_n-(-C\equiv C-)-(CH_2)_m-$,

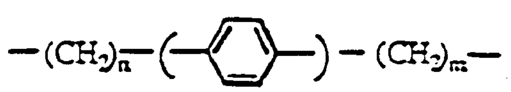

wherein n and m independently represent 1 to 10 providing that when x is O then neither n nor m is l, and further when x is l,A also represents

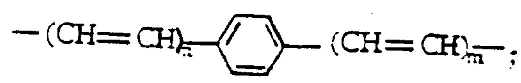

or a pharmaceutically acceptable salt thereof, which comprises reacting rapamycin with a diacyl halide of formula II

X-CO-A-COX (II)

wherein X is a halide and A is as defined in above and if desired isolating the product as a pharmaceutically acceptable salt thereof.

**2.** A process as claimed in Claim 1 wherein A is $-(CH_2)_n-$ in which n is 4 to 7, or A is

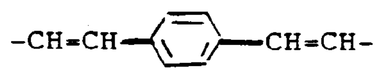

.

**3.** A process as claimed in Claim 1 wherein x is O and A is $-(CH_2)_4-$.
**4.** A process as claimed in Claim 1 wherein x is O and A is $-(CH_2)_5-$.
**5.** A process as claimed in Claim 1 wherein x is O and A is $-(CH_2)_6-$.
**6.** A process as claimed in Claim 1 wherein x is I and A is $-(CH_2)_4-$.
**7.** A process as claimed in Claim 1 wherein x is I and A is $-(CH_2)_5-$.
**8.** A process as claimed in Claim 1 wherein x is I and A is $-(CH_2)_6-$.
**9.** A process as claimed in Claim 1 wherein x is I and A is $-(CH_2)_7-$.
**10** A process as claimed in Claim 1 wherein x is I and A is

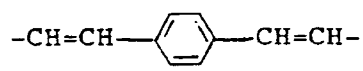

**11.** A process as claimed in any one of Claims 1 to 10 wherein X is chlorine.

**12.** A process for preparing a pharmaceutical composition which comprises bringing a compound of formula I as defined in Claim 1 into association with a pharmaceutically acceptable carrier.

European Patent Office

# EUROPEAN SEARCH REPORT

Application Number

EP 92 30 4645

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | JOURNAL OF THE AMERICAN CHEMICAL SOCIETY. vol. 113, no. 4, 13 February 1991, GASTON, PA US pages 1409 - 1411; H. FRETZ ET AL.: 'Rapamycin and FK506 binding proteins (Immunophilins)' * page 1410, Scheme I, compounds 2b,2c * | 1 | C07D498/22 C07D519/00 A61K31/445 //(C07D498/22, 321:00,311:00, 273:00,221:00) (C07D519/00, 498:00,498:00) |
| D,A | US-A-4 650 803 (V. J. STELLA ET AL.) * claims 1,5 * | 1,13 | |
| D,A | EP-A-0 046 661 (AYERST, MC KENNA & HARRISON) * claims 1,6 * | 1,13 | |
| | | | TECHNICAL FIELDS SEARCHED (Int. Cl.5 ) |
| | | | C07D A61K |

The present search report has been drawn up for all claims

| Place of search | Date of completion of the search | Examiner |
|---|---|---|
| THE HAGUE | 25 AUGUST 1992 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)